# EUROPÄISCHE PATENTSCHRIFT

(11) **EP 1 677 808 B1**
(45) Veröffentlichungstag und Bekanntmachung des Hinweises auf die Patenterteilung: **15.12.2010**
(21) Anmeldenummer: 04790770.4
(22) Anmeldetag: 22.10.2004
(51) Int. Cl.: A61K 33/04, A61K 38/28, A61K 31/436, A61K 31/573, A61P 31/12

(54) **KOMBINATIONSPRÄPARAT ZUR BEHANDLUNG DER SEPSIS**
COMBINED PREPARATION FOR TREATING SEPSIS
PREPARATION COMBINEE POUR TRAITER UNE SEPSIE

(30) Priorität: 22.10.2003 DE 10349115
(43) Veröffentlichungstag der Anmeldung: 12.07.2006
(73) Patentinhaber: Biosyn Arzneimittel GmbH, 70734 Fellbach (DE)
(72) Erfinder: STIEFEL, Thomas, 70184 Stuttgart (DE)
(74) Vertreter: Grünecker, Kinkeldey, Stockmair & Schwanhäusser Anwaltssozietät
(86) Internationale Anmeldenummer: PCT/EP2004/011978
(87) Internationale Veröffentlichungsnummer: WO 2005/039603

(56) Entgegenhaltungen:
- WO-A-00/12101
- US-A- 4 512 977
- DATABASE WPI Section Ch, Derwent Publications Ltd., London, GB; Class B04, AN 2002-563534 XP002313189 & KR 2002 012 947 A (JUNG S H) 6. Februar 2003 (2003-02-06)
- DATABASE WPI Section Ch, Week 199706 Derwent Publications Ltd., London, GB; Class B04, AN 1997-059687 XP002313314 & JP 08 308561 A (SUMITOMO ELECTRIC IND CO) 26. November 1996 (1996-11-26)
- DATABASE WPI Section Ch, Week 198323 Derwent Publications Ltd., London, GB; Class B01, AN 1983-55444K XP002313315 & RO 80 055 A (INTR ANTIBIOTICE) 30. Oktober 1982 (1982-10-30)
- SENECA H ET AL: "Glucocorticoid therapy in sepsis/shock caused by gram-negative microorganisms." JOURNAL OF THE AMERICAN GERIATRICS SOCIETY. NOV 1975, Bd. 23, Nr. 11, November 1975 (1975-11), Seiten 493-502, XP009042536 ISSN: 0002-8614

## Beschreibung

Die vorliegende Erfindung betrifft ein Kombinationspräparat zur Behandlung der Sepsis.

Sepsis, SIRS (systemic inflammatory response syndrome) und septischer Schock sind die Haupttodesursachen auf nicht-kardiologischen Intensivstationen. In den USA versterben nach Angaben des Center of Disease Control ca. 200.000 Menschen jährlich an den Folgen einer Sepsis, vergleichbar der Sterblichkeit bei akutem Herzinfarkt. Nach aktuellen Erhebungen aus den USA stieg die Zahl der Sepsisfälle von 1979 mit 82,7/100.000 auf 240/100.000 im Jahr 2000 an. Die Prävalenz der Sepsis in den USA wird auf ca. 600.000 pro Jahr geschätzt. Mit einer Inzidenz von ca. 300 auf 100.000 Einwohner ist die Sepsis eine häufigere Erkrankung als der Darmkrebs (50/100.000), Brustkrebs (110/100.000) oder Aids (17/100.000). Die Mortalität nahm in dem Zeitraum von 1979 bis 2000 von 27,8 % auf nur 17,9 % ab und somit stieg die Anzahl der an Sepsis verstorbenen Patienten in den letzten 20 Jahren signifikant an. Die geschätzten Krankenhauskosten betragen ca. 17 Mrd. US$. Trotz der Entwicklung sehr wirksamer Antibiotika hat sich durch ihren breiten Einsatz- die Mortalität bei Sepsispatienten nicht wesentlich beeinflussen lassen. Nicht die Mikroorganismen allein sind offenbar verantwortlich für den tödlichen Verlauf, sondern die Reaktion des Organismus auf die Infektion. Weitgehend Einigkeit besteht darin, dass die Überstimulation des Immunsystems durch die bei der Sepsis aktivierten Zytokine zum Multiorganversagen und Tod führt. Interventionsstudien, die die Folgereaktion von Zytokinen wie z. B TNFα blockieren, haben jedenfalls nicht zu einem verbesserten Überleben geführt.

Sepsis ist die Antwort des Körpers auf eine Infektion. Normalerweise bekämpft das körpereigene Abwehrsystem eine Infektion, aber bei schwerer Sepsis führt die Reaktion des Körpers zu einem Überschiessen und setzt eine Kaskade von Vorgängen in Lauf, die zu einer ausgedehnten Entzündung und Blutgerinnung in winzigen Gefäßen im gesamten Körper führt. Die Formen von Sepsis umfassen auch schwere Sepsis, die vorliegt, wenn eine akute Organfehlfunktion oder ein vollständiges Organversagen erfolgt; septischen Schock, der bei schwerer Sepsis entsteht, wenn das kardiovaskuläre System anfängt auszufallen, so dass der Blutdruck fällt und lebensnotwendige Organe nicht mehr mit einer ausreichenden Sauerstoffmenge versorgt werden.

Auslöser einer Sepsis kann jegliche Infektion - bakteriellen, viralen, parasitären Ursprungs oder durch Pilze verursacht - sein, wobei diese Infektion überall im Körper vorkommen kann. Sepsis kann jeden treffen in jedem Alter, obwohl der junge oder sehr alte Krankenhauspatient und Menschen mit bestehenden Krankheitszuständen ein größeres Risiko tragen. Risikofaktoren umfassen ein zu wenig aktives Immunsystem (wie es beispielsweise während einer Chemotherapie entstehen kann oder durch Medikamente verursacht wird, die eine Organtransplantation ermöglichen sollen; durch chirurgische Eingriffe; künstliche Beatmung; genetische Veranlagung oder bei invasiven Verfahren z. B. bei Zuführen von Flüssigkeiten.)

Sepsis ist die Antwort des Körpers auf eine Infektion. Die Symptome können folgende Anzeichen umfassen: Fieber und Schüttelfrost; verringerte mentale Aufmerksamkeit manchmal in Verbindung mit Verwirrtheit; Durchfall; erhöhte Pulsfrequenz (mehr als 90 Schläge pro Minute); erhöhte Atemfrequenz (mehr als 20 Atemzüge pro Minute); hohe oder niedrige Zahl an weißen Blutplättchen; niedriger Blutdruck; veränderte Nieren- oder Leberfunktion. Eine Sepsis kann sich rasch entwickeln. Je schneller sie diagnostiziert und behandelt wird, um so besser ist es. Die häufigsten Infektionsstellen, die zu Sepsis führen, sind die Lunge, der Darmtrakt, der Unterleib und das Becken. Bei bis zu 30 % der Patienten wird jedoch die genaue Infektionsursache nicht identifiziert. Der Verlauf der Krankheit kann häufig unvorhersagbar sein.

Der Verlauf der Sepsis kann als Abfolge von verschiedenen Vorgängen beschrieben werden. Wenn die Sepsis beginnt, reagiert der Körper mit ausgedehnten Entzündungen, Blutgerinnung und verschlechtertem Abbau von Blutgerinnseln.

Unter normalen Umständen werden Substanzen, die auch Immunmodulatoren genannt werden, freigesetzt, um den Körper bei dem Kampf gegen die Infektion bei dem Heilungsvorgang selbst zu unterstützen. Bei einer Person mit Sepsis bricht dieser Mechanismus zusammen und die Immunregulatoren führen zu einer überschiessenden Reaktion. Die Infektion fördert die Freisetzung von zu vielen dieser Regulatoren, die die Auskleidung der Blutgefäße entzünden und die Vorgänge zur Blutgerinnung werden aktiviert, wobei dieser Vorgang eine weitere Welle der Freisetzung von Regulatoren in Gang bringt. Die Entzündung führt zur Freisetzung einer Substanz, welche die Bildung von Blutgerinnseln stimuliert. Bei der Sepsiskaskade ist die Fähigkeit des Körpers, die Blutgerinnsel abzubauen, unterdrückt. Eine Substanz, die an der Bildung der Blutgerinnung, der Kontrolle der Entzündung und dem Abbau von Gerinnseln beteiligt ist, aktiviertes Protein C genannt, ist bei der Sepsis verringert. Als Ergebnis der Blutgerinnselbildung und der Unfähigkeit, diese Gerinnsel abzubauen, beginnen sich mikroskopische Blutgerinnsel in lebensnotwendigen Organen, Armen und Beinen, abzulagern und den Blutfluss zu beschränken, wobei dies zu Gewebeschäden führt, die zu einem Organversagen führen können.

Die Diagnose der Sepsis kann manchmal schwierig sein. Einige Symptome, wie Fieber, hoher Puls und Atemschwierigkeiten treten regelmäßig auf und können manchmal einem anderen Verursacher zugeordnet werden. Die erste Maßnahme bei der Behandlung ist das Identifizieren und Beseitigen der zugrundeliegenden Infektion mit infektionshemmenden Mitteln oder chirurgischen Eingriffen, um den Infektionsherd zu beseitigen. Abhängig von dem Zustand des Patienten können andere Behandlungen durchgeführt werden, wie das Verabreichen von Flüssigkeiten, Wirkstoffen zum Erhöhen des Blutdrucks, mechanische Beatmung, um die Atmung zu unterstützen oder die Dialyse bei Nierenversagen.

Bis zur jüngsten Vergangenheit hat kein einziges Mittel und keine Behandlungsstrategie ausreichende Wirkung für die routinemäßige Behandlung von Patienten mit Sepsis gezeigt.

So hat beispielsweise die Verwendung von Gluconaten, wie des Mg-Gluconats, den prinzipiellen Nachteil, dass diese in den Blutzucker-regulierenden Mechanismus des Körpers eingreifen. Daher sollte prinzipiell, aber insbesondere beim zusätzlichen Einsatz von Insulin die Verwendung solcher Substanzen bei der Sepsis/SIRS-Behandlung, vermieden werden.

Einige Ärzte vermuten, dass ein Wirkstoff, der aktiviertes Protein C erhöhen könnte, ein Schlüssel zu der erfolgreichen Behandlung der schweren Sepsis sein könnte, wenn das Sterberisiko sehr hoch ist.

So hat beispielsweise die Verwendung von Gluconaten, wie des Mg-Gluconats, den prinzipiellen Nachteil, dass diese in den Blutzucker-regulierenden Mechanismus des Körpers eingreifen. Daher sollte prinzipiell, aber insbesondere beim zusätzlichen Einsatz von Insulin die Verwendung solcher Substanzen bei der Sepsis/SIRS-Behandlung, vermieden werden.

Auch bilden freie Radikale ein mögliches Angriffsziel bei der Behandlung von Sepsis.

### Freie Radikale und Sepsis

Es ist bekannt, dass während einer systemischen Entzündung oder Sepsis Wasserstoffperoxyd und Superoxyde freigesetzt werden. Gleichzeitig aber sind die antioxidativen Mechanismen wie die Aktivität der Superoxyd-Dismutase, Glutathionperoxidase (GPx) und Katalase sowie die Konzentration von α-Tocopherol und Ascorbinsäure vermindert. Die vermehrte Expression der iNOS (inducible nitric oxide synthase) verursacht eine Vasodilatation und Translokation von NF-κB und damit die Transkription und Translatation einer Reihe von inflammatorischen Zytokinen. NO reagiert mit den Superoxyd-Radikalen zum gewebetoxischen Peroxynitrit. Die durch freie Radikale hervorgerufene Zellschädigung lässt sich an den erhöhten Spiegeln von konjugierten Dienen, Thiobarbitursäure-Reaktionsprodukten und Malondialdehyden bei SIRS und Sepsis nachweisen.

Eine adjuvante Therapie mit Antioxidanzien wie Ascorbinsäure, Glutathion, N-Acetyl-L-Cystein oder Vitamin A, E und C allein oder in Kombination kann die Morbidität bei Patienten mit schweren Verbrennungen reduzieren. Auch die Mikrozirkulation kann verbessert werden, die Lipidperoxidation vermindert, das Herzminutenvolumen gesteigert und damit die Volumensubstitution reduziert werden. Die Translokation von NF-κB ist geringer, dadurch werden weniger inflammatorische Zytokine wie TNFa, IL-1β und IL-6 freigesetzt. Die bei SIRS/Sepsis vermehrt gebildeten freien Radikale spielen also eine wesentliche Rolle in der Organschädigung, und eine Therapie mit Antioxidantien beeinflusst den natürlichen Verlauf einer Sepsis günstig durch Modulation der Immunreaktion.

Selenoenzyme, die Glutathionreduktasen (GPx) und Thioreduktasen sind die zentralen Enzymsysteme, die das Redox-Gleichgewicht sowohl plasmatisch, cytosolisch und im Zellkern aufrecht erhalten. Sie benötigen Selen zur Bildung der 21. Aminosäure, Selenocystein, das im aktiven Zentrum der Selenenzyme vorliegt.

Die Plasmaselenspiegel sind bei Patienten mit SIRS/Sepsis signifikant vermindert. Sie spiegeln zwar nicht den Selengehalt des Organismus wieder, die gleichzeitig verminderte plasmatische GPx-Aktivität aber zeigt, dass in der Sepsis offenbar der Bedarf an Selen gesteigert ist. Wenngleich auch bisher nicht gezeigt werden konnte, dass alle gewebsständigen, am Redox-system beteiligten Selenoenzyme vermindert sind, so konnte doch nachgewiesen werden, dass unter einer Selensubstitution die NF-κB Translokation vermindert ist, was darauf hinweist, dass offensichtlich weniger freie Radikale gebildet werden.

Das Wirkprinzip der Antioxidantien ist die Hemmung der Bildung (Deferoxamon, Alopurimol) oder die Bindung (Radikalfänger, N-Acetyl-Cystein, Dimethylsulfoxyd, Dimethylschwefelhamstoff) und der Abbau (Superoxyd-Dismutase, Katalase) von Sauerstoffradikalen.

Von N-Acetyl-Cystein konnte gezeigt werden, dass es bei Patienten mit einer sich entwickelnden respiratorischen Insuffizienz im Rahmen der Sepsis zu einer Verbesserung der pulmonalen Funktion sowie auch der Verbesserung der radiologischen Veränderungen führt.

Beim Einsatz von Vitamin E wird die Lipidperoxidation in der Sepsis reduziert. Verminderte Vitamin E-Serumspiegel wurden in Sepsis induzierten ARDS-Fällen häufiger nachgewiesen.

Auch die Gabe von Vitamin C vermag die Serumlipidperoxyde drastisch zu reduzieren. In jüngster Zeit werden insbesondere Tocopherol und Ascorbinsäure als mögliche Wirkstoffe in der Sepsis untersucht.

JP 08 308561 A (SUMITOMO ELECTRIC IND CO) und KR2002012947 offenbaren Selenhaltige Kultivierungsmedien für Zellen, aber keine pharmazeutischen Verbindungen.

RO 80 055 A (INTR ANTIBIOTICE) und US4512977 offenbaren Selen-haltige Salben und Gele, aber keine wässrigen Lösungen.

WO0012101 offenbart die Behandlung von SIRS mit Selen und erwähnt die mögliche Kombination mit anti-inflammatorischen Verbindungen zur SIRS Behandlung.

SENECA H ET AL, JOURNAL OF THE AMERICAN GERIATRICS SOCIETY. NOV 1975, Bd. 23, Nr. 11, November 1975 (1975-11), Seiten 493-502 offenbart die Behandlung von Sepsis mit Glukokortikoiden.

Der vorliegenden Erfindung lag das technische Problem zugrunde, ein weiteres pharmazeutisches Präparat anzugeben, das bei der Behandlung der Sepsis bzw. der SIRS bzw. septischem Schock eine Verbesserung der bestehenden Therapien ermöglicht. Insbesondere war es ein Ziel, ein Mittel anzugeben, das die beträchtlich hohe Morbidität bei Sepsisfällen zu senken vermag.

Dieses technische Problem wird erfindungsgemäß gelöst durch eine pharmazeutische Zusammensetzung, enthaltend eine Wirkstoffkombination, die einen Selen-haltigen Wirkstoff und ein Kortikoid umfasst, wobei die Wirkstoffe in wässrige Lösung vorliegen. Im Einzelnen offenbart die Erfindung
I. Pharmazeutische Zusammensetzung, enthaltend eine Wirkstoffkombination umfassend einen Selen-haltigen Wirkstoff und ein Kortikoid, wobei die Wirkstoffe in wässriger Lösung vorliegen.
II. Verwendung einer Wirkstoffkombination, umfassend einen selenhaltigen Wirkstoff und ein Kortikoid zur Herstellung eines Medikaments zur Behandlung von Sepsis, SIRS und/oder sepsischem Schock.

Dabei müssen die genannten Wirkstoffe nicht zwingend in einer Verabreichungsform gemeinsam vorliegen, sondern können als Einzelformulierungen eingesetzt werden. Dabei kann die Verabreichung der beiden Wirkstoffe entweder zeitgleich oder zeitlich gestaffelt erfolgen. Bei einer bevorzugten Ausführungsform wird die Wirkstoffkombination aus Selen und Kortikoid zusätzlich ergänzt um Insulin, wobei Insulin eine unterstützende Funktion dergestalt herbeiführt, dass eine strenge Blutzuckereinstellung bei Patienten mit schwerer Sepsis einen insgesamt positiven Verlauf auf die Krankheit nimmt. Dieser Einsatz von Insulin kann auch als supportive Therapie zu der eigentlichen Therapie mit den Wirkstoffen Selen und Kortikoid angesehen werden.

Die Formulierung der Wirkstoffe zu einem geeigneten Präparat ist dem Fachmann geläufig und kann beispielsweise auch European Pharmacopoeia, 4th Edition, Supplement 4.6, herausgegeben vom EDQM, 2003 entnommen werden. Auch lassen sich die Wirkstoffe, wie sie bereits als Einzelpräparate aus dem Handel bekannt sind, ohne weiteres zu der erfindungsgemäßen Wirkstoffkombination zusammenfügen.

Die Wirkstoffe liegen jeweils in wässriger Lösung vor, wobei diese Lösung vorzugsweise für die i. V.-Appfikation der Wirkstoffe geeignet sein sollten.

Geeignete Konzentrationen für den selenhaltigen Wirkstoff liegen in dem Bereich von 5 - 500 µg/ml Selen. Zu beachten Ist jedoch, dass sich diese Angaben auf den tatsächlichen Gewichtsanteil des Selens beziehen, der natürlich beispielsweise bei Natriumselenit geringer ist als er dies bei reinem Selen wäre. So entsprechen 50 µg Selen 0,167 mg Natriumselenit x 5H₂O

Der selenhaltige Wirkstoff wird vorzugsweise ausgewählt aus pharmazeutisch verträglichen Selensalzen, wobei als besonders bevorzugter selenhaltiger Wirkstoff Natriumselenit eingesetzt wird.

Bei einer weiteren bevorzugten Ausführungsform wird das Kortikoid ausgewählt aus Glucocorticoiden.

Als besonders bevorzugte Ausführungsform enthält die erfindungsgemäße pharmazeutische Zusammensetzung Hydrocortison (Kortisol) als Kortikoidbestandteil.

Die Konzentration des Kortikoidbestandteils liegt vorzugsweise in dem Bereich von 0.5 - 50 mg/ml. Besonders bevorzugt ist bevorzugt ist 5 mg Hydrocortison/ml in 50% Ethanol-Wasser Gemisch (v/v).

Entsprechende Hilfsstoffe, Träger, Verdünnungsmittel, etc. der pharmazeutischen Zusammensetzung wird der Fachmann auf dem Gebiet je nach Verabreichungsform wählen bzw. abstimmen. Solche pharmazeutischen Zusammensetzungen unterscheiden sich von Hydrocortisonzusammensetzungen, die z.B. in der Form von topisch verabreichbaren Präparaten in der Augenheilkunde eingesetzt werden.

Die erfindungsgemäße Wirkstoffkombination wird vorzugsweise eingesetzt zur Behandlung der Sepsis, SIRS oder dem septischen Schock. Als "Sepsis" wird insbesondere die systemische Reaktion auf eine Infektion angesehen, die charakterisiert ist durch zwei oder mehr der folgenden Symptome:
a. Körperkerntemperatur > 38°C oder < 36°C,
b. Herzfrequenz > 90/min.,
c. Atemfrequenz > 20/min. oder PaCO₂ < 32 mm Hg (Spontanatmung),
d. Leukozyten > 12.000/mm³ oder < 4.000/mm³, oder > 10 % unreife (stabförmige) Formen,
wobei sich bei "schwerer Sepsis" zusätzlich eine Organdysfunktion, Minderperfusion oder Hypotonie einstellt. Die Minderdurchblutung bzw. Durchblutungsstörungen können eine Lactatazidose, Oligurie oder eine akute Änderung der Bewusstseinslage beinhalten. Zu diesem Krankheitsbild gehört auch das "Sepsissyndrom", da ebenfalls durch eine systemisch entzündliche Reaktion auf eine Infektion hin gekennzeichnet ist und zwei oder mehr der folgenden Symptome aufweist:
a. Körperkerntemperatur > 38°C oder < 36°C,
b. Herzfrequenz > 90/min,
c. Atemfrequenz > 20/min. oder PaCO₂ < 32 mm Hg (Spontanatmung)
d. Mindestens eines der nachfolgend genannten Zeichen unzureichender Organfunktion/Organperfusion:
   ° veränderte zerebrale Funktion (gestörte Bewusstseinslage)
   ° PaO₂ < 75 mm Hg (bei Raumluft, keine COPD vorhanden)
   ° erhöhte Serum-Laktatkonzentration
   ° vermindertes HZV: < 30 ml/h oder < 0,5 ml/kg* über mehr als 1 h

Unter "SIRS" versteht man eine systemisch-entzündliche Reaktion auf verschiedene schwere klinische Insulte hin, die ebenfalls durch zwei oder mehr der folgenden klinischen Symptome charakterisiert sind:
a. Körperkerntemperatur > 38°C oder < 36°C,
b. Herzfrequenz > 90/min.,
c. Atemfrequenz > 20/min. oder PaCO₂ < 32 mm Hg (Spontanatmung),
d. Leukozyten > 12.000/mm³ oder > 4.000/mm³, oder 10 % unreife (stabförmige) Formen.

Schließlich handelt es sich beim "septischen Schock" um einen Sepsis-induzierten Schock mit Hypotonie trotz adäquater Volumensubstitution eingehend mit Durchblutungsstörungen. Eine Sonderform stellt der "refraktäre septische Schock" dar, wobei es sich hierbei um einen septischen Schock ohne rasches Ansprechen auf intravenöse Volumengabe, (auf zum Beispiel 500 ml Plasmaexpander in 30 Minuten) und Vasopressor (zum Beispiel Dopamin mehr als 10 µg/kg pro Minute) handelt.

Bei einer bevorzugten Ausführungsform werden mindestens 100 µg, vorzugsweise mindestens 500 µg Selen (entsprechend zum Beispiel 1,67 mg Natriumselenit x 5 H₂O) pro Tag verabreicht. Bei einer besonders bevorzugten Ausführungsform werden mindestens 3,34 mg Natriumselenit pro Tag verabreicht (entsprechend 1000 µg Selen).

Eine bevorzugte Verabreichungsform für das Selen ist die Verabreichung mittels einer einmaligen Bolusinjektion pro Tag.

Weitere bevorzugte Verabreichungsformen sind die parentarale (i.v.), wie die orale Verabreichung. Wo es die Umstände verlangen, wird der Fachmann des weiteren zur enteralen Verabreichung (z.B. via Magen- oder Darmsonde) greifen.

Bei einer weiteren bevorzugten Ausführungsform erfolgt die Verabreichung des selenhaltigen Wirkstoffs für eine Dauer von mindestens 7 Tage, vorzugsweise für eine Dauer von mindestens 14 Tage.

Bei einer weiteren bevorzugten Ausführungsform erfolgt eine zusätzliche Basisapplikation an Selen, beispielsweise mindestens 20 µg, vorzugsweise mindestens 35 µg Natriumselenit pro Tag. Diese zusätzliche Basisapplikation dient bei der totalen parenteralen Ernährung zum Ausgleich des üblichen Verlustes.

Bei einer weiteren bevorzugten Ausführungsform werden mindestens 50 mg Hydrocortison pro Tag verabreicht, wobei eine Menge von 200 mg Hydrocortison pro Tag besonders bevorzugt ist.

Bei einer weiteren bevorzugten Ausführungsform wird das Cortison kontinuierlich über einen Zeitraum von 24 Stunden verabreicht. Dies kann beispielsweise mittels typischer Infusionslösungen erfolgen.

Bei einer weiteren bevorzugten Ausführungsform erfolgt die Verabreichung des Hydrocortisons mit den oben angegebenen Tagesdosen über einen Zeitraum von mindestens 2, vorzugsweise mindestens 5, besonders bevorzugt mindestens 14 Tagen oder so lange, bis die Sepsis überwunden ist.

Schließlich ist es bevorzugt, die oben erläuterte Kombinationstherapie mit Selen und Kortikoid durch eine supportive Therapie mit Insulin zu ergänzen, die insbesondere den Blutzuckerspiegel so einstellen soll, dass 200 mg% nicht überschritten werden.

Die vorliegenden Untersuchungen haben eine Abnahme der Mortalität bei Behandlung von Patienten mit dem erfindungsgemäßen pharmazeutischen Präparat gezeigt. Es wurde die Abnahme der Mortalität von Patienten mit schwerer Sepsis, die entweder nur mit Natriumselenit oder nur mit Hydrocortison oder gänzlich ohne diese beiden Wirkstoffe behandelt wurden, untersucht, wobei alle Gruppen jedoch als supportive Therapie Insulin erhielten. Die ermittelten Daten zeigen eindeutig, dass die Gruppe, die sowohl Selen wie auch Hydrocortison erhielt, eine deutlich verringerte Mortalität aufwies, die über den rein additiven Effekt der Selen- und Hydrocortisonwirkung hinausgeht. Vielmehr zeigen die Daten eine überraschende synergistische Wirkung dieser beiden Wirkstoffe hinsichtlich der Verringerung der Mortalität. Die Patienten erhielten entweder 1000 µg Selen pro Tag oder 200 mg Hydrocortison pro Tag, bzw. eine Kombination davon. Die Zahlen in den Säulen zeigen die Anzahl der Patienten pro Gruppe.

Die folgenden Beispiele erläutern die Erfindung.

Erfindungsgemäßer Therapieansatz bei schwerer Sepsis:

Die vorliegende Erfindung beruht auf einer prospektiven randomisierten Studie bei Patienten mit schwerer Sepsis und einem Apache III Score von über 70 Punkten. Es wurde untersucht, inwieweit sich die Mortalität bei diesen Patienten mit einer Kombinationstherapie aus Natriumselenit und Hydrocortison, begleitet von einer strengen Blutzuckereinstellung mittels Insulin, reduzieren lässt. Die Patienten wurden randomisiert und geblindet, entweder mit Natriumselenit, in Form eines 1000 µg Bolus pro Tag, gefolgt von weiteren täglichen Bolusinjektionen mit jeweils 1000 µg Natriumselenit für 14 Tage bzw. Placebo-behandelt. Zusätzlich erhielten die Patienten als Basis 35 µg Natriumselenit pro Tag. Alle Patienten erhielten zusätzlich Hydrocortison, 200 mg kontinuierlich über 24 Stunden, verabreicht; dies über die gesamte Dauer der schweren Infektion. Die weitere Medikation einschließlich der Verabreichung von Antibiotika entsprach der gängigen Praxis. Zusätzlich wurde der Blutzucker mit Insulin so eingestellt, dass er unter 200 mg% lag. Das Ergebnis war eine Reduktion der Mortalität bei den nur mit Selen-behandelten Patienten um etwa 10-20%, bei den nur mit Hydrocortison-behandelten Patienten ebenfalls um 10-20%, aber bei der Kombination Hydrocortison/Natriumselenit konnte die Mortalität bei schwerkranken, septischen Patienten mit der Kombinationstherapie (Natriumselenit, Hydrocortison und Blutzuckerkontrolle) um 80 % gesenkt werden. Dieses Ergebnis zeigt eindeutig den erzielbaren synergistischen Effekt bei der Kombinationstherapie mit Natriumselenit und Hydrocortison bei gleichzeitiger strenger Blutzuckereinstellung mit Insulin.

## Patentansprüche

1. Pharmazeutisches Kombinationspräparat, enthaltend eine Wirkstoffkombination umfassend einen selenhaltigen Wirkstoff und ein Kortikoid, wobei die Wirkstoffe in wässriger Lösung vorliegen.

2. Pharmazeutisches Kombinationspräparat nach Anspruch 1, **dadurch gekennzeichnet, dass** die Wirkstoffkombination weiterhin Insulin umfasst.

3. Pharmazeutisches Kombinationspräparat nach einem der Ansprüche 1 oder 2, **dadurch gekennzeichnet, dass** die Wirkstoffe jeweils separat in getrennten Verabreichungsformen vorliegen.

4. Pharmazeutisches Kombinationspräparat nach einem der Ansprüche 1 bis 3, **dadurch gekennzeichnet, dass** jeder Wirkstoff in einer für die i. v.-Applikation geeigneten Form vorliegt.

5. Pharmazeutisches Kombinationspräparat nach einem der Ansprüche 1 bis 4, **dadurch gekennzeichnet, dass** die Konzentration des Selens in dem Bereich von 5 - 500 µg/ml, vorzugsweise bei 50 µg/ml liegt und die Konzentration des Kortikoids in dem Bereich von 0,5-50 mg/ml, vorzugsweise 5 mg/ml, liegt.

6. Pharmazeutisches Kombinationspräparat nach einem der Ansprüche 1 bis 5, **dadurch gekennzeichnet, dass** das Selen in einer Form vorliegt, ausgewählt aus pharmazeutisch verträglichen Selensalzen.

7. Pharmazeutisches Kombinationspräparat nach Anspruch 6, **dadurch gekennzeichnet, dass** der selenhaltige Wirkstoff als Natriumselenit, vorzugsweise Natrium-Selenit x 5H₂O, vorliegt.

8. Pharmazeutisches Kombinationspräparat nach einem der Ansprüche 1 bis 7, **dadurch gekennzeichnet, dass** das Kortikoid ausgewählt wird aus Glucocorticoiden.

9. Pharmazeutisches Kombinationspräparat nach Anspruch 8, **dadurch gekennzeichnet, dass** das Kortikoid Hydrocortison ist.

10. Verwendung einer Wirkstoffkombination, umfassend einen selenhaltigen Wirkstoff und ein Kortikoid zur Herstellung eines Medikaments zur Behandlung von Sepsis, SIRS und/oder septischem Schock.

11. Verwendung nach Anspruch 10, **gekennzeichnet durch** eine Wirkstoffkombination wie in einem der Ansprüche 1 bis 9 definiert.

12. Verwendung nach Anspruch 10 oder 11, **dadurch gekennzeichnet, dass** mindestens 100 µg, vorzugsweise mindestens 1000 µg, Selen pro Tag verabreicht werden.

13. Verwendung nach Anspruch 11 oder 12, **dadurch gekennzeichnet, dass** mindestens 3340 µg Natriumselenit x 5H₂O pro Tag verabreicht werden.

14. Verwendung nach einem der Ansprüche 11 oder 13, **dadurch gekennzeichnet, dass** die Verabreichung des Selen-haltigen Wirkstoffs mittels eines Bolus einmal pro Tag erfolgt.

15. Verwendung nach einem der Ansprüche 11 bis 14, **dadurch gekennzeichnet, dass** die Verabreichung des selenhaltigen Wirkstoffs über eine Dauer von mindestens 7 Tage, vorzugsweise mindestens 14 Tage, erfolgt.

16. Verwendung nach einem der Ansprüche 10 bis 15, **dadurch gekennzeichnet, dass** eine zusätzliche Basisapplikation von mindestens 20 µg, vorzugsweise mindestens 35 µg. Natriumselenit x 5H₂O pro Tag erfolgt.

17. Verwendung nach einem der Ansprüche 10 bis 16, **dadurch gekennzeichnet, dass** mindestens 50 mg, vorzugsweise mindestens 200 mg, Hydrocortison pro Tag verabreicht werden.

18. Verwendung nach Anspruch 10 oder 11, **dadurch gekennzeichnet, dass** das Hydrocortison über 24 Stunden kontinuierlich verabreicht wird.

19. Verwendung nach einem der Ansprüche 17 oder 18, **dadurch gekennzeichnet, dass** das die Hydrocortisonbehandlung mindestens 2, vorzugsweise mindestens 5 Tage lang erfolgt.

20. Verwendung nach einem der Ansprüche 10 bis 19, **dadurch gekennzeichnet, dass** zusätzlich Insulin verabreicht wird, so dass der Blutzucker 11 mmol/l nicht übersteigt.

21. Verwendung eines selenhaltigen Wirkstoffs in der Therapie von Sepsis, SIRS und/oder septischem Schock mit Hydrocortison.

22. Verwendung nach einem der Ansprüche 10 bis 21, wobei der selenhaltige Wirkstoff und das Kortikoid in Einzelformulierungen verabreicht werden, entweder zeitgleich oder zeitlich gestaffelt.

## Claims

1. Combined pharmaceutical preparation, containing an active substance combination comprising a selenium-containing active substance and a corticoid, wherein the active substances are present in an aqueous solution.

2. Combined pharmaceutical preparation as claimed in claim 1, **characterised in that** the active substance combination also comprises insulin.

3. Combined pharmaceutical preparation as claimed in any one of claims 1 or 2, **characterised in that** the active substances are each present separately in separate forms of administration.

4. Combined pharmaceutical preparation as claimed in any one of claims 1 to 3, **characterised in that** each active substance is present in a form suitable for IV-application.

5. Combined pharmaceutical preparation as claimed in any one of claims 1 to 4, **characterised in that** the concentration of selenium is in the range of 5 - 500 µg/ml, preferably 50 µg/ml, and the concentration of the corticoid is in the range of 0.5 - 50 mg/ml, preferably 5 mg/ml.

6. Combined pharmaceutical preparation as claimed in any one of claims 1 to 5, **characterised in that** the selenium is present in a form selected from pharmaceutically compatible selenium salts.

7. Combined pharmaceutical preparation as claimed in claim 6, **characterised in that** the selenium-containing active substance is present as sodium selenite, preferably sodium-selenite x 5H₂O.

8. Combined pharmaceutical preparation as claimed in any one of claims 1 to 7, **characterised in that** the corticoid is selected from glucocorticoids.

9. Combined pharmaceutical preparation as claimed in claim 8, **characterised in that** the corticoid is hydrocortisone.

10. Use of an active substance combination, comprising a selenium-containing active substance and a corticoid to produce a drug for the treatment of sepsis, SIRS and/or septic shock.

11. Use as claimed in claim 10, **characterised by** an active substance combination as defined in any one of claims 1 to 9.

12. Use as claimed in claim 10 or 11, **characterised in that** at least 100 µg, preferably at least 1000 µg, of selenium is administered per day.

13. Use as claimed in claim 11 or 12, **characterised in that** at least 3340 µg sodium selenite x 5H₂O is administered per day.

14. Use as claimed in any one of claims 11 or 13, **characterised in that** the selenium-containing active substance is administered by means of a bolus once per day.

15. Use as claimed in any one of claims 11 to 14, **characterised in that** the selenium-containing active substance is administered over a period of at least 7 days, preferably at least 14 days.

16. Use as claimed in any one of claims 10 to 15, **characterised in that** at least 20 µg, preferably at least 35 µg, sodium selenite x 5H₂O is additionally administered as a basis application per day.

17. Use as claimed in any one of claims 10 to 16, **characterised in that** at least 50 mg, preferably at least 200 mg, hydrocortisone is administered per day.

18. Use as claimed in claim 10 or 11, **characterised in that** the hydrocortisone is administered continuously over 24 hours.

19. Use as claimed in any one of claims 17 or 18, **characterised in that** the hydrocortisone treatment is performed for at least 2 days, preferably for at least 5 days.

20. Use as claimed in any one of claims 10 to 19, **characterised in that** insulin is additionally administered, so that blood sugar does not exceed 11 mmol/l.

21. Use of a selenium-containing active substance in the treatment of sepsis, SIRS and/or septic shock with hydrocortisone.

22. Use as claimed in any one of claims 10 to 21, wherein the selenium-containing active substance and the corticoid are administered in individual formulations, either simultaneously or at time intervals.

## Revendications

1. Association médicamenteuse pharmaceutique contenant une association de principes actifs comprenant un principe actif à teneur en sélénium et un corticoïde, dans laquelle les principes actifs sont présents en solution aqueuse.

2. Association médicamenteuse pharmaceutique selon la revendication 1, **caractérisée en ce que** l'association de principes actifs comprend en outre de l'insuline.

3. Association médicamenteuse pharmaceutique selon l'une quelconque des revendications 1 ou 2, **caractérisée en ce que** les principes actifs sont respectivement présents séparément, dans des formes d'administration individuelles.

4. Association médicamenteuse pharmaceutique selon l'une quelconque des revendications 1 à 3, **caractérisée en ce que** chaque principe actif est présent sous une forme appropriée pour une application par voie intraveineuse.

5. Association médicamenteuse pharmaceutique selon l'une quelconque des revendications 1 à 4, **caractérisée en ce que** la concentration du sélénium se situe dans la plage de 5 à 500 µg/ml, de préférence de 50 µg/ml, et la concentration du corticoïde se situe dans la plage de 0,5 à 50 mg/ml, de préférence de 5 mg/ml.

6. Association médicamenteuse pharmaceutique selon l'une quelconque des revendications 1 à 5, **caractérisée en ce que** le sélénium se présente sous une forme choisie parmi les sels de sélénium acceptables au plan pharmaceutique.

7. Association médicamenteuse pharmaceutique selon la revendication 6, **caractérisée en ce que** le principe actif à teneur en sélénium se présente sous la forme de sélénite de sodium, de préférence, de sélénite de sodium x 5H₂O.

8. Association médicamenteuse pharmaceutique selon l'une quelconque des revendications 1 à 7, **caractérisée en ce que** le corticoïde est choisi parmi les glucocorticoïdes.

9. Association médicamenteuse pharmaceutique selon la revendication 8, **caractérisée en ce que** le corticoïde est l'hydrocortisone.

10. Utilisation d'une association de principes actifs, comprenant un principe actif à teneur en sélénium et un corticoïde pour la fabrication d'un médicament destiné au traitement d'une septicémie, d'un SRIS et/ou d'un choc septique.

11. Utilisation selon la revendication 10, **caractérisée par** une association de principes actifs, telle que définie dans l'une quelconque des revendications 1 à 9.

12. Utilisation selon la revendication 10 ou 11, **caractérisée en ce que** l'on administre au moins 100 µg, de préférence au moins 1000 µg de sélénium par jour.

13. Utilisation selon la revendication 11 ou 12, **caractérisée en ce que** l'on administre au moins 3340 µg de sélénite de sodium x 5H₂O par jour.

14. Utilisation selon l'une quelconque des revendications 11 ou 13, **caractérisée en ce que** l'administration du principe actif à teneur en sélénium est effectué au moyen d'un bolus à raison d'une fois par jour.

15. Utilisation selon l'une quelconque des revendications 11 à 14, **caractérisée en ce que** l'administration du principe actif à teneur en sélénium est effectuée sur période de temps d'au moins 7 jours, de préférence d'au moins 14 jours.

16. Utilisation selon l'une quelconque des revendications 10 à 15, **caractérisée en ce que** l'on procède à une application de base supplémentaire d'au moins 20 µg, de préférence d'au moins 35 µg de sélénite de sodium x 5H₂O par jour.

17. Utilisation selon l'une quelconque des revendications 10 à 16, **caractérisée en ce que** l'on administre au moins 50 mg, de préférence au moins 200 mg d'hydrocortisone par jour.

18. Utilisation selon la revendication 10 ou 11, **caractérisée en ce que** l'hydrocortisone est administrée sur une période de 24 heures en mode continu.

19. Utilisation selon l'une quelconque des revendications 17 ou 18, **caractérisée en ce que** le traitement à base d'hydrocortisone est effectué sur une période d'au moins 2, de préférence, d'au moins 5 jours.

20. Utilisation selon l'une quelconque des revendications 10 à 19, **caractérisée en ce que** l'on administre en outre de l'insuline de sorte que la quantité de sucre dans le sang ne dépasse pas 11 mmoles/l.

21. Utilisation d'un principe actif à teneur en sélénium dans la thérapie de la septicémie, du SRIS et/ou du choc septique, avec de l'hydrocortisone.

22. Utilisation selon l'une quelconque des revendications 10 à 21, dans laquelle le principe actif à teneur en sélénium et le corticoïde sont administrés sous la forme de formulations individuelles, de manière simultanée ou décalée dans le temps.
